(19) Europäisches Patentamt | European Patent Office | Office européen des brevets

(11) **EP 2 588 664 B1**

# (12) EUROPEAN PATENT SPECIFICATION

(45) Date of publication and mention
of the grant of the patent:
**18.01.2017 Bulletin 2017/03**

(21) Application number: **11800017.3**

(22) Date of filing: **29.06.2011**

(51) Int Cl.:
*D21C 3/20* (2006.01)  *C08H 7/00* (2011.01)
*C08H 8/00* (2010.01)  *D21C 3/00* (2006.01)
*D21C 3/04* (2006.01)  *D21C 3/22* (2006.01)
*C12P 7/10* (2006.01)  *C12P 19/00* (2006.01)
*C13K 1/02* (2006.01)  *D21C 7/10* (2006.01)

(86) International application number:
**PCT/CA2011/000760**

(87) International publication number:
**WO 2012/000093 (05.01.2012 Gazette 2012/01)**

## (54) ORGANOSOLV PROCESS

ORGANOSOLV-VERFAHREN

TRAITEMENT À BASE D'ORGANOSOLV

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2010 US 360377 P**

(43) Date of publication of application:
**08.05.2013 Bulletin 2013/19**

(73) Proprietor: **Fibria Innovations Inc.
British Columbia V7X 1T2 (CA)**

(72) Inventors:
• **BERLIN, Alex
Burnaby
Briish Columbia V5E 3M9 (CA)**
• **BALAKSHIN, Mikhail, Y.
North Vancouver
British Columbia V7N 4C1 (CA)**
• **MA, Raymond
Burnaby
British Columbia V5G 3L1 (CA)**
• **MAXIMENKO GUTMAN, Vera
Burnaby
British Columbia V5E 3M9 (CA)**
• **ORTIZ, Darwin
Burnaby
British Colombia V5G 3L1 (CA)**

(74) Representative: **Roberts, Michael Austin
Reddie & Grose LLP
16 Theobalds Road
London WC1X 8PL (GB)**

(56) References cited:
**CA-A1- 2 611 152    US-A- 4 594 130
US-B1- 7 465 791**

**Description**

FIELD

This disclosure relates to an organosolv biorefining process.

BACKGROUND

For environmental, economic, and resource security reasons, there is an increasing desire to obtain energy and material products from bio-renewable resources and particularly from "waste" and/or non-food biomass feedstocks. The various chemical components within typical biomass can be employed in a variety of ways. In particular, the cellulose and hemicellulose in plant matter may desirably be separated out and fermented into fuel grade alcohol. And the lignin component, which makes up a significant fraction of species such as trees and agricultural waste, has huge potential as a useful source of aromatic chemicals for numerous industrial applications. However, most separation techniques employed by industry today are not optimal for providing industrially useful chemicals. For example, the techniques may be too harsh and chemically alter the lignin component during separation to the point where it is no longer acceptable for use in many of these potential applications.

Organosolv extraction processes can be used to separate lignin and other useful materials from biomass. Such processes can be used to capitalize on the value from multiple components in the biomass. Organosolv extraction processes however typically involve extraction with a volatile solvent at higher temperatures and pressures than other industrial methods and thus are generally more complex and expensive. While large scale commercial viability had been demonstrated decades ago from a technical and operational perspective, organosolv extraction has not, to date, been widely adopted.

SUMMARY

The present invention provides in one aspect an organosolv "biorefining" process comprising:

a) Adding the biomass to a reaction vessel and exposing the biomass to a solvent wherein:

   i. the ratio of solvent to biomass is from about 10:1 to about 4:1;
   ii. the solvent comprises from 40 to 60% w/w ethanol;
   iii. from about 1.5% to about 2.5% (based on dry weight of the biomass) of an acid is added;

b) Elevating the temperature of the biomass/solvent mixture to from about 140°C to about 170°C for a total period of from about 50 minutes to about 200 minutes to form a pulp and an extraction liquor said liquor comprising derivatives of native lignin;
c) Recovering at least a portion of the extraction liquor;
d) Recovering at least a portion of the pulp;

wherein the pressure in the reaction vessel is about 18 bar or less, and
wherein step a) produces pretreated biomass solids with a Time-to-Conversion-Target (TCT) equal to 120 h or less and an Overall Glucose Conversion (OGC) of 50% or higher.

In a further aspect of the invention, there is provided an organosolv process for treating a lignocellulosic biomass comprising:

a) Adding the biomass to a reaction vessel and exposing the biomass to a solvent wherein:

   i. the ratio of solvent to biomass is from about 10:1 to about 4:1;
   ii. the solvent comprises from 40 to 60% w/w ethanol;
   iii. the initial pH of the biomass/solvent mixture is from about 1.5 to about 2.5;

b) Elevating the temperature of the biomass/solvent mixture to from about 140°C to about 170°C for a total period of from about 50 minutes to about 200 minutes to form a pulp and an extraction liquor said liquor comprising derivatives of native lignin;
c) Recovering the extraction liquor;
d) Recovering the pulp;

wherein the pressure in the reaction vessel is about 18 bar or less, and

wherein step a) produces pretreated biomass solids with a Time-to-Conversion-Target (TCT) equal to 120 h or less and an Overall Glucose Conversion (OGC) of 50% or higher.

In the organosolv processes of the invention, the pressure in the reaction vessel may be about 16 bar or less.

The elevated temperature of step (b) may be from about 150°C to about 165°C.

The elevated temperature of step (b) may be maintained for about 120 minutes to about 180 minutes.

The solvent may comprise from 50% to 60% w/w ethanol.

In the organosolv processes of the invention, from about 2% to about 2.5% (based on dry weight of the biomass) of an acid may be added.

The pH of the biomass/solvent mixture may be from about 1.6 to about 2.3.

The ratio of solvent to biomass may be from about 9:1 to about 7:1.

The biomass may be comprise softwood feedstock, hardwood feedstock, annual fibre feedstock, or a combination thereof.

The yield of lignin recovered from the extraction liquor may be 60%, 70%, 80% or greater of the theoretical maximum yield.

At least a portion of the recovered pulp may be converted into carbohydrates which are subsequently converted into ethanol.

The present processes thus comprise treating a lignocellulosic biomass in the presence of a solvent and under certain conditions to separate at least a part of the lignin from the biomass.

As used herein, the term "biorefining" refers to the co-production of bio-based products (e.g. lignin derivatives), fuel (e.g. ethanol), and/or energy from biomass.

As used herein, the term "organosolv" refers to bio-refinery processes wherein the biomass is subject to an extraction step using solvent at an elevated temperature.

As used herein, the term "native lignin" refers to lignin in its natural state, in plant material.

As used herein, the terms "lignin derivatives" and "derivatives of native lignin" refer to lignin material extracted from lignocellulosic biomass. Usually, such material will typically be a mixture of chemical compounds that are generated during the extraction process.

Advantages of the invention will be apparent to those of ordinary skill in the art upon review of the following description of specific embodiments of the invention.

BRIEF DESCRIPTION OF THE DRAWINGS

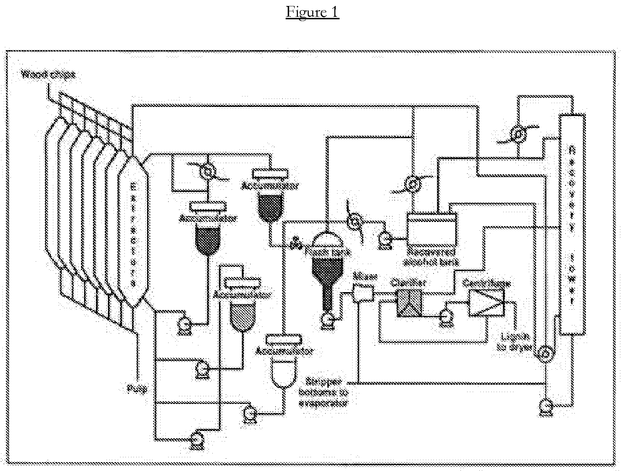

Figure 1 shows an example of a Lignol® lignin (Alcell®) organosolv process;
Figure 2 shows the dependence of lignin & glucose yields on solids content (L:W Ratio) at 2% acid on aspen wood, 50% EtOH in liquor, 120 min cooking time, 165 °C cooking temperature;
Figures 3, 4 and 5 shows the time-to-conversion target for various biomass samples.

DETAILED DESCRIPTION

The present disclosure provides an organosolv biorefining process. Organsolv processes are well known in the art. See, for example, US Patent 4,100,016; US Patent 4,764,596; US Patent 5,681,427; US Patent 7,465,791; US Patent Application 2009/0118477; US Patent Application 2009/0062516; US Patent Application 2009/00669550; or US Patent 7,649,086.

Four major "organosolv" pulping processes have been tested on a trial basis. The first method uses ethanol/water pulping (aka the Lignol® (Alcell®) process); the second method uses alkaline sulphite anthraquinone methanol pulping (aka the "ASAM" process); the third process uses methanol pulping followed by methanol, NaOH, and anthraquinone pulping (aka the "Organocell" process); the fourth process uses acetic acid/hydrochloric acid or formic acid pulping (aka the "Acetosolv" and "Formacell" processes).

A description of the Lignol® Alcell® process can be found, for example, in US Patent 4,764,596 or Kendall Pye and Jairo H. Lora, The Alcell™ Process, Tappi Journal, March 1991, pp. 113-117 (the documents are herein incorporated by reference). The process generally comprises pulping or pre-treating a fibrous biomass feedstock with primarily an ethanol/water solvent solution under conditions that include: (a) 60% ethanol/40% water (W/W), (b) a temperature of about 180° C to about 210° C, and (c) pressure of about 20 atm to about 35 atm. Derivatives of native lignin are fractionated from the biomass into the pulping liquor which also receives solubilised hemicelluloses, other carbohydrates and other components such as resins, phytosterols, terpenes, organic acids, phenols, and tannins. Organosolv pulping liquors comprising the fractionated derivatives of native lignin and other components from the fibrous biomass feedstocks, are often called "black liquors". The organic acid and other components released by organosolv pulping significantly acidify the black liquors to pH levels of about 5 and lower. After separation from the pre-treated lignocellulosic biomass or pulps produced during the pre-treatment process (e.g. pulping process), the derivatives of native lignin are recovered from the black liquor by flashing followed by dilution with acidified cold water and/or stillage which will cause most of the fractionated derivatives of native lignin to precipitate thereby enabling their recovery by standard solids/liquids separation processes. Various disclosures exemplified by US Patent No. 7,465,791 and PCT Patent Application Publication No. WO 2007/129921, describe modifications to the Lignol® Alcell® organosolv.

Organosolv processes, particularly the Lignol® Alcell® process, can be used to separate highly purified lignin derivatives and other useful materials from biomass. Such processes may therefore be used to exploit the potential value of the various components making up the biomass.

Organosolv extraction processes however typically involve extraction with a volatile solvent at higher temperatures and pressures compared to other industrial processes and thus are generally considered to be more complex and expensive. For example, when the processes are run at higher pressures (~25-30 bar) capital costs can increase due to the necessity of using more robust equipment. In addition, the necessity of heating the biomass to high temperatures requires extra expense in terms of energy input leading to increased operating costs.

Moreover, organosolv extraction processes can result in the production of self-precipitated lignins or lignins with poor solubility in the cooking liquor (SPLs), particularly when using softwood biomass but also when other types of biomass is used. SPLs can attach to metal surfaces causing equipment to be fouled and are difficult to remove.

In order to improve the commercial viability of organosolv processes it is desirable to keep capital and operating costs low while maximizing the potential revenue streams. For example, the cost of the enzymes used to convert the cellulose-rich pulp to mono- and/or oligosaccharides which can then be fermented into biofuels such as ethanol and n-butanol or bio-based chemicals such as xylitol and other sugar-alcohols, succinic acid and other organic acids, etc represents a significant operating cost and, therefore, it would be advantageous to reduce the amount of enzymes needed. Also, recovered lignin derivatives represent a source of high-value chemicals and, therefore, it would be advantageous to increase the yield of such substances.

Surprisingly, it has been found that organosolv processes operated within relatively narrow ranges of process conditions offer significant advantages in terms of, for example, glucose yield and/or lignin derivative yield.

The present disclosure offers an organosolv process which operates at significantly lower temperature and pressure than typical for organosolv biorefining allowing, for example, savings in capital, operating, and/or energy expenditure.

Embodiments of the present process demonstrate significantly less equipment fouling than seen in prior art organosolv processes. For example, when the present process utilizes softwood feedstock there is a marked reduction in the amount of SPLs seen. A reduction in the amount of SPLs can result in lower equipment fouling. This offers the possibility of an improved commercial scale organosolv plant that has the ability to process softwood and other types of biomass that suffer from problems with SPLs.

Typical organosolv processes, such as Lignol's® Alcell® process, generally recover around 60% of the theoretical maximum lignin. The remaining lignin content is generally degraded and ends up as a waste residue. This non-recovered fraction can be toxic to microorganisms and can contaminate certain of the product streams reducing their processability by microorganisms and/or value.

Embodiments of the present disclosure offer surprisingly high lignin yields which increases the value derivable from the lignin stream of a particular process and may also reduce the amount of non-recovered lignin contaminating product streams from the process.

Embodiments of the present disclosure offer pretreated solids ("pulps") with surprisingly good enzymatic hydrolyzability. This characteristic increases the pulps reactivity to enzymes and, hence, reduces the amount of enzyme needed for converting the pulp to sugars and subsequently to ethanol or other chemicals.

Embodiments of the present disclosure offer surprisingly high yields of glucose.

The present invention in generic terms provides an organosolv process comprising:

(a) pretreating (e.g. pulping) a lignocellulosic biomass with an organic solvent to form a pulp comprising cellulose and an extraction liquor comprising lignin derivatives;
(b) separating the cellulosic pulp from the extraction liquor; and
(c) recovering at least a portion of the extracted compounds from the extraction liquor.

At least a portion of the cellulosic pulp may be converted into carbohydrates, ethanol, or other chemicals.

The pretreatment step (a) of the present process can be operated at pressures of about 18 bar or less. For example, about 17 bar or less, about 16 bar or less, about 15 bar or less.

The biomass/solvent mixture of pretreatment step (a) of the present process may be heated to a temperature of from about 150°C or greater, about 152°C or greater, about 154°C or greater, up to a temperature of from about 170°C or less, about 168°C or less, about 166°C or less, about 165°C or less.

For example, the biomass/solvent mixture of pretreatment step (a) of the present process may be heated to a temperature of from about 155°C to about 165°C.

The biomass/solvent mixture of pretreatment step (a) of the present process may be kept at the elevated temperature for about 50 minutes or more, about 55 minutes or more, about 60 minutes or more, about 65 minutes or more, about 70 minutes or more, about 75 minutes or more, about 80 minutes or more, about 95 minutes or more, about 100 minutes or more, about 105 minutes or more, about 110 minutes or more, about 115 minutes or more, about 120 minutes or more, up to about 200 minutes or less, about 195 minutes or less, about 190 minutes or less, about 185 minutes or less, about 180 minutes or less.

For example, the biomass/solvent mixture of pretreatment step (a) of the present process may be kept at the elevated temperature for about 120 to about 180 minutes.

The solvent mixture of pretreatment step (a) of the present process may comprise about 40% or more, about 42% or more, about 44% or more, about 46% or more, about 48% or more, about 50% or more, up to about 60% or less, about 58% or less, about 56% or less, ethanol as organic solvent.

For example, the solvent mixture of pretreatment step (a) of the present process may comprise about 45% to about 60%, about 50% to about 55%, ethanol.

The solvent mixture of pretreatment step (a) of the present process may have a pH of from about 1.5 or greater, about 1.6 or greater, about 1.7 or greater. The solvent mixture of pretreatment step (a) of the present process may have a pH of from about 2.5 or lower, about 2.4 or lower, about 2.3 or lower. For example, the solvent mixture of pretreatment step (a) of the present process may have a pH of from about 1.5 to about 2.5. For example, from about 1.6 to about 2.3.

From about 1.5% or greater, about 1.7% or greater, about 1.9% or greater, about 2% or greater, by weight, of acid (based on dry weight wood) may be added to the biomass. From about 3% or lower, about 2.7% or lower, about 2.5% or lower, by weight, of acid (based on dry weight wood) may be added to the biomass.

The weight ratio of liquor to biomass in the pretreatment step (a) may be from about 10:1 to about 4:1, about 9:1 to about 5:1, about 8:1 to about 6:1.

The pretreatment step (a) of the present process may generate pretreated biomass solids with Time-to-Conversion-Target (TCT) equal to about 120 h or less, about 110 h or less, about 100 h or less, about 90 h or less, about 80 h or less, about 75 h or less, about 60 h or less, about 40 h or less. The pretreatment step (a) may generate pretreated biomass solids with an Overall Glucose Conversion (OGC) of about 50% or higher, about 65% or higher, about 70% or higher, about 75% or higher, about 80% or higher, about 85% or higher.

The present organic solvent comprising ethanol may further comprise any suitable solvent. For example, aromatic alcohols such as phenol, catechol, and combinations thereof; short chain primary and secondary alcohols, such as methanol, propanol, and combinations thereof. For example, the solvent may be a mix of ethanol & water.

The present process may utilize any suitable lignocellulosic feedstock including hardwoods, softwoods, annual fibres, energy crops, municipal waste, and combinations thereof.

Hardwood feedstocks include Acacia; Afzelia; *Synsepalum duloificum;* Albizia; Alder (e.g. *Alnusglutinosa, Alnus rubra*); Applewood; Arbutus; Ash (e.g. *F. nigra*, *F. quadrangulata*, *F. excelsior*, *F. pennsylvanica lanceolata*, *F. latifolia*, *F. profunda*, *F. americana*); Aspen (e.g. *P. grandidentata, P. tremula, P. tremuloides*); Australian Red Cedar (*Toona ciliata*); Ayna *(Distemonanthus benthamianus)*; Balsa (*Ochroma pyramidale*); Basswood (e.g. *T. americana, T. heterophylla*); Beech (e.g. *F. sylvatica*, *F. grandifolia*); Birch; (e.g. *Betula populifolia, B. nigra, B. papyrifera, B. lenta, B. alleghaniensis/B. lutea, B. pendula, B. pubescens*); Blackbean; Blackwood; Bocote; Boxelder; Boxwood; Brazilwood; Bubinga; Buckeye (e.g. *Aesculus hippocastanum, Aesculus glabra, Aesculus flava/ Aesculus octandra*); Butternut; Catalpa; Cherry (e.g. *Prunus serotina, Prunus pennylvanica, Prunus avium*); Crabwood; Chestnut; Coachwood; Cocobolo; Corkwood; Cottonwood (e.g. *Populus balsamifera, Populus deltoides, Populus sargentii, Populus heterophylla*); Cucumbertree; Dogwood (e.g. *Cornus florida, Cornus nuttallii*); Ebony (e.g. *Diospyros kurzii, Diospyros melanida, Diospyros crassiflora*); Elm (e.g. *Ulmus americana, Ulmus procera, Ulmus thomasii, Ulmus rubra, Ulmus glabra);* Eucalyptus; Greenheart; Grenadilla; Gum (e.g. *Nyssa sylvatica, Eucalyptus globulus, Liquidambar styraciflua, Nyssa aquatica*); Hickory (e.g. *Carya alba, Carya glabra, Carya ovata, Carya laciniosa*); Hornbeam; Hophornbeam; Ipê; Iroko; Ironwood (e.g. Bangkirai, *Carpinus caroliniana, Casuarina equisetfolia, Choricbangarpia subargentea, Copaifera* spp., *Eusideroxylon zwageri, Guajacum officinale, Guajacum sanctum, Hopea odorata,* Ipe, Krugiodendron *ferreum, Lyonothamnus lyonii (L. floribundus)*, *Mesua ferrea, Olea* spp., *Olneya tesota, Ostrya virginiana, Parrotia persica, Tabebuia serratifolia);* Jacarandá; Jotoba; Lacewood; Laurel; Limba; Lignum vitae; Locust (e.g. *Robinia pseudacacia, Gleditsia triacanthos*); Mahogany; Maple (e.g. *Acer saccharum, Acer nigrum, Acer negundo, Acer rubrum, Acer saccharinum, Acer pseudoplatanus);* Meranti; Mpingo; Oak (e.g. *Quercus macrocarpa, Quercus alba, Quercus stellata, Quercus bicolor, Quercus virginiana, Quercus michauxii, Quercus prinus, Quercus mublenbergii, Quercus chrysolepis, Quercus lyrata, Quercus robur, Quercus petraea, Quercus rubra, Quercus velutina, Quercus laurifolia, Quercus falcata, Quercus nigra, Quercus phellos, Quercus texana*); Obeche; Okoumé; Oregon Myrtle; California Bay Laurel; Pear; Poplar (e.g. P. balsamifera, P. nigra, Hybrid Poplar (*Populus × canadensis*)); Ramin; Red cedar; Rosewood; Sal; Sandalwood; Sassafras; Satinwood;

Silky Oak; Silver Wattle; Snakewood; Sourwood; Spanish cedar; American sycamore; Teak; Walnut (e.g. *Juglans nigra*, *Juglans regia);* Willow (e.g. *Salix nigra*, *Salix alba*); Yellow poplar (*Liriodendron tulipifera*); Bamboo; Palmwood; and combinations/hybrids thereof.

For example, hardwood feedstocks for the present invention may be selected from Acacia, Aspen, Beech, Eucalyptus, Maple, Birch, Gum, Oak, Poplar, and combinations/hybrids thereof. The hardwood feedstocks for the present invention may be selected from *Populus* spp. (e.g. *Populus tremuloides), Eucalyptus* spp. (e.g. *Eucalyptus globulus*), *Acacia* spp. (e.g. *Acacia dealbata*), and combinations/hybrids thereof.

Softwood feedstocks include Araucaria (e.g. *A. cunninghamii, A. angustifolia, A. araucana*); softwood Cedar (e.g. *juniperus virginiana, Thuja plicata, Thuja occidentalis, Chamaecyparis thyoides Callitropsis nootkatensis*); Cypress (e.g. *Chamaecyparis, Cupressus Taxodium, Cupressus arizonica, Taxodium distichum, Chamaecyparis obtusa, Chamaecyparis lawsoniana, Cupressus semperviren*); Rocky Mountain Douglas fir; European Yew; Fir (e.g. *Abies balsamea, Abies alba, Abies procera, Abies amabilis*); Hemlock (e.g. *Tsuga canadensis, Tsuga mertensiana, Tsuga heterophylla*); Kauri; Kaya; Larch (e.g. *Larix decidua, Larix kaempferi, Larix laricina, Larix occidentalis*); Pine (e.g. *Pinus nigra, Pinus banksiana, Pinus contorta, Pinus radiata, Pinus ponderosa, Pinus resinosa, Pinus sylvestris, Pinus strobus, Pinus monticola, Pinus lambertiana, Pinus taeda, Pinus palustris, Pinus rigida, Pinus echinata*); Redwood; Rimu; Spruce (e.g. *Picea abies, Picea mariana, Picea rubens, Picea sitchensis, Picea glauca*); Sugi; and combinations/hybrids thereof.

For example, softwood feedstocks which may be used herein include cedar; fir; pine; spruce; and combinations/hybrids thereof. The softwood feedstocks for the present invention may be selected from loblolly pine (*Pinus taeda*), radiata pine, jack pine, spruce (e.g., white, interior, black), Douglas fir, *Pinus silvestris, Picea abies,* and combinations/hybrids thereof. The softwood feedstocks for the present invention may be selected from pine (e.g. *Pinus radiata, Pinus taeda*); spruce; and combinations/hybrids thereof.

Annual fibre feedstocks include biomass derived from annual plants, plants which complete their growth in one growing season and therefore must be planted yearly. Examples of annual fibres include: flax, cereal straw (wheat, barley, oats), sugarcane bagasse, rice straw, corn stover, corn cobs, hemp, fruit pulp, alfalfa grass, esparto grass, switchgrass, and combinations/hybrids thereof. Industrial residues like corn cobs, fruit peals, seeds, etc. may also be considered annual fibres since they are commonly derived from annual fibre biomass such as edible crops and fruits. For example, the annual fibre feedstock may be selected from wheat straw, corn stover, corn cobs, sugar cane bagasse, and combinations/hybrids thereof.

The present invention will be further illustrated in the following examples. However it is to be understood that these examples are for illustrative purposes only.

EXAMPLES

The following examples are intended to be exemplary of the invention and are not intended to be limiting.

Example 1:

All the modeling work was performed with the help of two software packages: Microsoft Excel 2007 & MatLab Version 7.7.0.471 (R2008b) with Model-Based Calibration Toolbox Version 3.5 & the CAGE Optimization Module (The MathWorks, Inc., MA, USA).

The aspen chips used for the optimization were produced by Econotech after debarking and splitting logs sourced from a local BC forest. Validation of the found optimal region was performed with aspen chips supplied from Slave Lake, Alberta and screened at Lignol by Pilot Plant Operations.

Enzymatic hydrolysis was run at 50 g scale at 16% solids, 120 h, 150 rpm, pH 5.0, CellicCTec2 loaded at 12 mg/g glucan. Samples were taken after 24 h hydrolysis but here for simplicity we will report only yields after 120 h hydrolysis. This experimental design has proven to be representative of what one can see at larger scale (4-L & 20-L fermentor scale).

Thirty eight sets of five process variables (Table 1) were selected to run the optimization experiments and the results were used to build the models (Table 3).

The produced models showed that one can find pretreatment conditions for Aspen biomass where the Lignin Yield is higher than 80% and the Glucose Yield is higher than 85%. In all studied conditions the operating pressure was around 16 bar or lower.

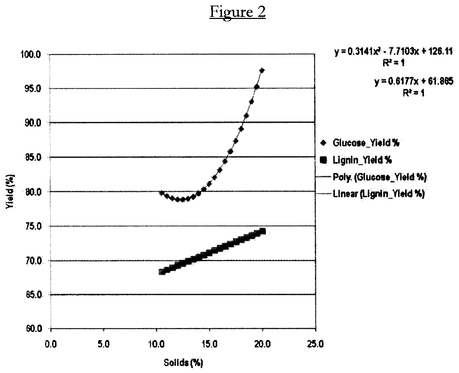

The optimum conditions for aspen lignin yield and glucose yield (~80% or higher theoretical yields) lies between 155 and 165 °C, ~50-55% EtOH, 120-180 min cooking time, 2.0-2.5% acid at a fixed L:W ratio of 8:1 to 7:1. Any combination of these conditions yields operating pressures around or below 16 bar. Decrease of L:W ratio (increase of % solids) is beneficial and leads to increase glucose and lignin yields under certain conditions such as the ones described in Figure 2.

Table 1 Sets of process variables selected to run the optimization experiments

| # | Acid | Time | Temperature | Ethanol | Solids | L:W |
|---|------|------|-------------|---------|--------|-----|
|   | % wt. | min | °C | %wt. | % | :1 |
| 1* | 2.00 | 105 | 148 | 55 | 15.0 | 5.7 |
| 2* | 2.50 | 143 | 139 | 48 | 12.5 | 7.0 |
| 3* | 1.50 | 68 | 156 | 63 | 17.5 | 4.7 |
| 4* | 1.38 | 133 | 158 | 53 | 10.6 | 8.4 |
| 5* | 1.63 | 114 | 145 | 42 | 11.9 | 7.4 |
| 6* | 2.06 | 166 | 144 | 63 | 10.3 | 8.7 |
| 7* | 1.69 | 175 | 151 | 50 | 17.2 | 4.8 |
| 8* | 2.44 | 82 | 155 | 62 | 10.9 | 8.1 |
| 9* | 2.00 | 112 | 152 | 55 | 13.1 | 6.6 |
| 10* | 1.41 | 168 | 152 | 57 | 12.7 | 6.9 |
| 11* | 2.50 | 145 | 143 | 48 | 11.6 | 7.6 |
| 12* | 2.28 | 140 | 150 | 63 | 12.0 | 7.3 |
| 13* | 1.50 | 78 | 161 | 63 | 14.6 | 5.8 |
| 14* | 1.78 | 103 | 159 | 55 | 14.5 | 5.9 |
| 15* | 2.53 | 121 | 137 | 59 | 10.8 | 8.3 |
| 16* | 1.97 | 145 | 149 | 43 | 10.5 | 8.6 |
| 17* | 2.75 | 128 | 139 | 66 | 13.9 | 6.2 |
| 18* | 1.75 | 62 | 157 | 51 | 10.9 | 8.2 |
| 19* | 1.59 | 98 | 143 | 53 | 11.1 | 8.0 |
| 20* | 1.25 | 95 | 148 | 59 | 15.4 | 5.5 |
| 21* | 1.34 | 79 | 164 | 41 | 12.3 | 7.1 |
| 22* | 2.83 | 92 | 139 | 63 | 13.8 | 6.2 |
| 23* | 2.25 | 161 | 166 | 44 | 12.4 | 7.1 |
| 24* | 1.58 | 111 | 152 | 67 | 10.1 | 8.9 |
| 25* | 2.08 | 73 | 161 | 59 | 12.6 | 7.0 |
| 26* | 1.88 | 170 | 136 | 61 | 15.0 | 5.7 |
| 27* | 1.20 | 45 | 159 | 69 | 12.0 | 7.4 |
| 28* | 2.45 | 176 | 137 | 65 | 13.2 | 6.6 |
| 29* | 2.88 | 103 | 154 | 46 | 12.0 | 7.4 |
| 30* | 1.52 | 162 | 140 | 60 | 15.4 | 5.5 |
| 31* | 2.27 | 69 | 144 | 49 | 11.6 | 7.6 |
| 32* | 2.38 | 70 | 145 | 68 | 13.5 | 6.4 |
| 33* | 2.89 | 153 | 134 | 51 | 11.0 | 8.1 |
| 34* | 1.89 | 78 | 151 | 66 | 16.0 | 5.2 |
| 35* | 2.14 | 172 | 155 | 47 | 12.3 | 7.2 |
| 36* | 1.38 | 136 | 163 | 53 | 10.5 | 8.5 |

(continued)

| # | Acid | Time | Temperature | Ethanol | Solids | L:W |
|---|---|---|---|---|---|---|
| | % wt. | min | °C | %wt. | % | :1 |
| 37* | 1.64 | 59 | 164 | 55 | 14.8 | 5.8 |
| 38* | 2.89 | 176 | 166 | 69 | 17.5 | 4.7 |
| Min | 1.20 | 45 | 134 | 41 | 10.1 | 4.7 |
| Max | 2.89 | 176 | 166 | 69 | 17.5 | 8.9 |

Table 2 Output boundaries of dependent process variables

| | Lignin Yield | Glucose Yield | Washed Pulp Yield |
|---|---|---|---|
| | % | % | % bd wood |
| Min | 51.5 | 70.2 | 42.8 |
| Max | 85.1 | 87.5 | 55.8 |

Table 3 Lignin Yield, Glucose Yield, and Washed Pulp Yields Models

**Lignin Yield (%) =** -146.0292+27.99547*Solids+0.03120071*Temp*EtOH-0.1121524*Temp*Solids+0.0015453*Time*Time+0.003185002*Time*EtOH+0.03481116*Time*Acid-0.04457228*Time*Solids-0.03969131*EtOH*EtOH-0.07047815*EtOH*Solids **($r^2$=0.705)**

**Glucose Yield (%) =** -62.07805+2.045008*Time-186.731*Acid+32.86724*Solids-0.007077812*Temp*Time+0.02055919*Temp*EtOH+0.7270062*Temp*Acid-0.1413414*Temp*Solids-0.005416981*Time*EtOH-0.05171966*Time*Solids+0.4617057*EtOH*Acid-0.2913332*EtOH*Solids+8.300157*Acid*Acid+1.758393*Acid*Solids+0.3140979*Solids*Solids **($r^2$=0.858)**

**Washed Pulp Yield (%) =** -15.8281+0.621621*EtOH-5.53252*Acid+15.7276*Solids+0.00179991*Temp*Temp-0.0821519*Temp*Solids+0.00062543*Time*Time-0.00132139*Time*EtOH+0.0230202*Time*Acid-0.0118592*Time*Solids-0.0444829*EtOH*Solids **($r^2$=0.901)**

**Example 2:**

Reactivity of biomass samples

"Time-to-Conversion-Target" (TCT, h) is a metric which characterizes biomass reactivity and it is defined as the time in hours required to enzymatically convert 85% of the total glucan in a pretreated biomass sample to monomeric glucose under the following reaction conditions:

12 mg protein/g glucan of the state-of-the-art enzyme CellicCTec2 (Novozymes North America Inc., Franklinton, NC, USA). The protein content in the preparation is determined by the Plerce® Micro BCA Protein Assay Kit (Thermo Fisher Scientific Inc., Waltham, MA, USA) in absence of interfering compounds or the enzyme protein content value is supplied by the enzyme manufacturer;

50 g total reaction weight;

16% total pretreated biomass solids in reaction;

pH 5.0, 0.1 M sodium citric buffer prepared in deionized water;

0.50 ppm antibiotic Lactrol®;

50 °C;

150 rpm mixing rate in an air incubator;

Five Zr beads per flask (Cat. No. 08-412-15C, Grinding Media for Ball Mills (Zirconia), O.D. x H 13x13 mm);

250-mL total volume of a sterilized by autoclaving glass Erlenmeyer reaction flask

The flask must be plugged with a foam plug cover by an aluminum foil to avoid evaporation or equivalent. The glucose released is measured chromatographically.

Table 4: Comparison of biomass reactivity (TCT, h) between three differently pretreated aspen samples

| Biomass Sample | $P_{max}$ (bar) | Acid (%wt) | pH | Time (min) | T (°C) | EtOH (%wt) | Solids (%wt) | L:W Ratio | TCT (h) |
|---|---|---|---|---|---|---|---|---|---|
| S10005865 28(1) | 9.7 | 1.20 | 2.20 | 45 | 159 | 69 | 12 | 7.40 | 168.6 |
| 14(1) | 12.8 | 1.50 | 2.02 | 78 | 161 | 63 | 15 | 5.85 | 121.5 |
| S1000563615(1) | 11.0 | 1.80 | 1.90 | 103 | 159 | 55 | 15 | 5.90 | 117.5 |

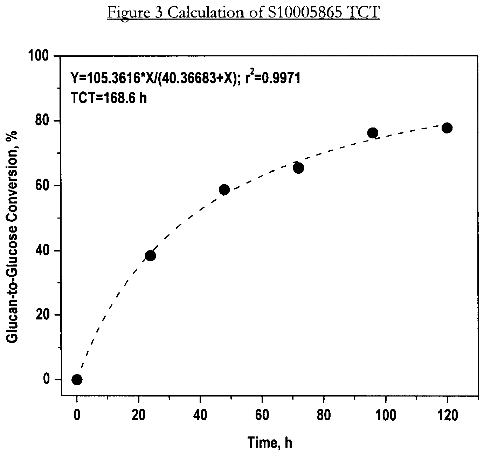

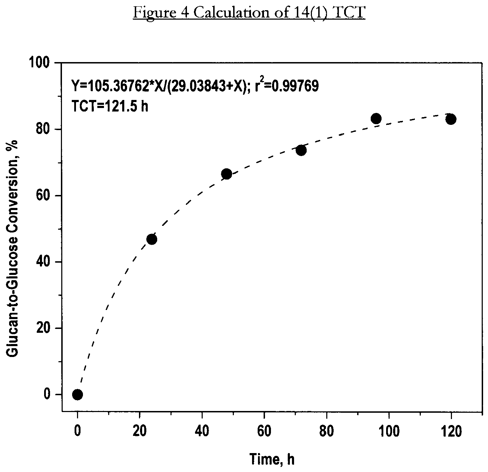

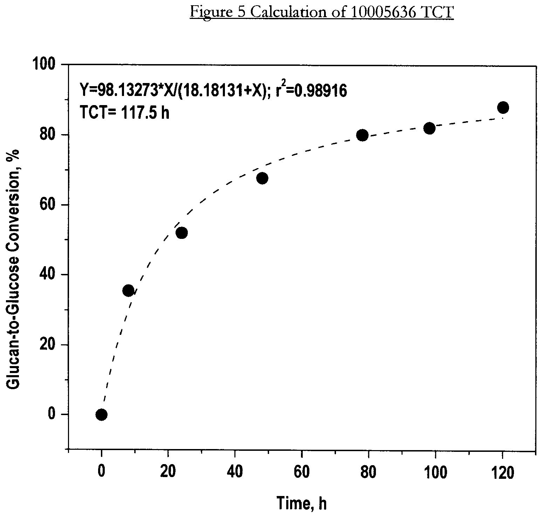

The pretreated biomass sample S10005636 15(1) shows the highest reactivity with the shortest time (117.5 h) required to achieve the target (85% glucan-to-glucose conversion) while the sample S10005865 28(1) shows the lowest reactivity with a 168.6 h TCT. The TCT values are calculated by extra- or intrapolation using the experimental hyperbolic functions Glucan-to-Glucose Conversion (%) vs. Time (h) (figures 3-5). These hyperbolic functions are typical of enzymatic hydrolysis reactions.

"Overall Glucose Conversion" (OGC, % total glucose in raw biomass) is a metric which provides the total glucose recovered from the pretreated solids in fermentable monomeric form and it integrates both the glucose recovery yield after biomass pretreatment (PGY - Pretreatment Glucose Yield) and the glucose hydrolysis yield after enzymatic hydrolysis (HGY - Hydrolysis Glucose Yield). The OGC is calculated as follows:

$$OGC\ (\%) = \frac{Recovered\_Glucose\_After\_Pretreatment\_per\_100g}{Pretreated\_Raw\_Material\ (g) * HGY(\%)}$$

Table 5: Overall Glucose Conversion (OGC, % total glucose in raw biomass) yields of three differently pretreated aspen samples

| Biomass Sample | $P_{max}$ (bar) | Acid (%wt) | pH | Time (min) | T (°C) | EtOH (%wt) | Solids (%wt) | L:W Ratio | OGC (%) |
|---|---|---|---|---|---|---|---|---|---|
| S10005865 28(1) | 9.7 | 1.20 | 2.20 | 45 | 159 | 69 | 12 | 7.40 | 78.17 |
| 14(1) | 12.8 | 1.50 | 2.02 | 78 | 161 | 63 | 15 | 5.85 | 76.57 |
| S10005636 15(1) | 11.0 | 1.80 | 1.90 | 103 | 159 | 55 | 15 | 5.90 | 79.11 |

"Maximum Operating Pressure" ($P_{max}$, bar) is defined as the maximum operating pressure reached during the biomass pretreatment stage. In the case of the present invention this value is around 16 bar or lower.

"Best Pretreated Biomass" (BPB) is defined as the pretreated biomass produced under $P_{max}$ around or lower than 16 bar which shows the lowest TCT and the highest OGC with the highest lignin yield. The lignin yield must be considered for economic reasons but it does not necessarily impacts biomass reactivity.

In the case of the three compared pretreated aspen samples the BPB is the sample S10005636 15(1) since it showed the highest OGC and the lowest TCT while the maximum operating pressure (11 bar) was kept well below the allowed maximum of 16 bar.

**Claims**

1. An organosolv process for treating a lignocellulosic biomass comprising:

   a) Adding the biomass to a reaction vessel and exposing the biomass to a solvent wherein:

      i. the ratio of solvent to biomass is from about 10:1 to about 4:1;
      ii. the solvent comprises from 40 to 60% w/w ethanol;
      iii. from about 1.5% to about 2.5% (based on dry weight of the biomass) of an acid is added;

   b) Elevating the temperature of the biomass/solvent mixture to from about 140°C to about 170°C for a total period of from about 50 minutes to about 200 minutes to form a pulp and an extraction liquor said liquor comprising derivatives of native lignin;
   c) Recovering at least a portion of the extraction liquor;
   d) Recovering at least a portion of the pulp;

   wherein the pressure in the reaction vessel is about 18 bar or less, and
   wherein step a) produces pretreated biomass solids with a Time-to-Conversion-Target (TCT) equal to 120 h or less and an Overall Glucose Conversion (OGC) of 50% or higher.

2. An organosolv process for treating a lignocellulosic biomass comprising:

   a) Adding the biomass to a reaction vessel and exposing the biomass to a solvent wherein:

      i. the ratio of solvent to biomass is from about 10:1 to about 4:1;
      ii. the solvent comprises from 40 to 60% w/w ethanol;
      iii. the initial pH of the biomass/solvent mixture is from about 1.5 to about 2.5;

   b) Elevating the temperature of the biomass/solvent mixture to from about 140°C to about 170°C for a total period of from about 50 minutes to about 200 minutes to form a pulp and an extraction liquor said liquor comprising derivatives of native lignin;
   c) Recovering the extraction liquor;
   d) Recovering the pulp;

   wherein the pressure in the reaction vessel is about 18 bar or less, and
   wherein step a) produces pretreated biomass solids with a Time-to-Conversion-Target (TCT) equal to 120 h or less and an Overall Glucose Conversion (OGC) of 50% or higher.

3. The process of claim 1 or 2 wherein the pressure in the reaction vessel is about 16 bar or less.

4. The process of claim 1 or 2 wherein the elevated temperature of step (b) is from about 150°C to about 165°C.

5. The process of claim 1 or 2 wherein the elevated temperature of step (b) is maintained for about 120 minutes to about 180 minutes.

6. The process of claim 1 or 2 wherein the solvent comprises from 50% to 60% w/w ethanol.

7. The process of claim 1 or 2 wherein from about 2% to about 2.5% (based on dry weight of the biomass) of an acid is added.

8. The process of claim 1 or 2 wherein the pH of the biomass/solvent mixture is from about 1.6 to about 2.3.

9. The process of claim 1 or 2 wherein the ratio of solvent to biomass is from about 9:1 to about 7:1.

10. The process of claim 1 or 2 wherein the biomass comprises softwood feedstock, hardwood feedstock, annual fibre feedstock, or a combination thereof.

11. The process of claim 1 or 2 wherein the yield of lignin recovered from the extraction liquor is 60% or greater of the theoretical maximum yield.

12. The process of claim 1 or 2 wherein the yield of lignin recovered from the extraction liquor is 70% or greater of the theoretical maximum yield.

13. The process of claim 1 or 2 wherein the yield of lignin recovered from the extraction liquor is 80% or greater of the theoretical maximum yield.

14. The process of claim 1 or 2 wherein at least a portion of the recovered pulp is converted into carbohydrates which are subsequently converted into ethanol.

**Patentansprüche**

1. Organosolv-Verfahren zum Behandeln einer Lignocellulosebiomasse, das Folgendes beinhaltet:

a) Geben der Biomasse in ein Reaktionsgefäß und Inkontaktbringen der Biomasse einem Lösungsmittel, wobei:

i. das Verhältnis zwischen Lösungsmittel und Biomasse bei etwa 10:1 bis etwa 4:1 liegt;
ii. das Lösungsmittel 40 bis 60 % w/w Ethanol enhält;
iii. etwa 1,5 % bis etwa 2,5 % (bezogen auf das Trockengewicht der Biomasse) einer Säure zugegeben werden;

b) Erhöhen der Temperatur des Biomasse/Lösungsmittel-Gemischs auf etwa 140°C bis etwa 170°C für eine Gesamtdauer von etwa 50 Minuten bis etwa 200 Minuten, um einen Zellstoff und eine Extraktionsbrühe zu bilden, wobei die genannte Brühe Derivate von nativem Lignin beinhaltet:
c) Gewinnen wenigstens eines Teils der Extraktionsbrühe;
d) Gewinnen wenigstens eines Teils des Zellstoffs;

wobei der Druck im Reaktionsgefäß etwa 18 bar oder weniger beträgt, und
wobei Schritt a) vorbehandelte Biomassefeststoffe mit einem Time-to-Conversion-Target (TCT - Zeit-bis-Umwandlungs-Ziel) von 120 h oder weniger und einer Overall Glucose Conversion (OGC - Gesamtglucoseumwandlung) von 50 % oder mehr hervorbringt.

2. Organosolv-Verfahren zum Behandeln einer Lignocellulosebiomasse, das Folgendes beinhaltet:

a) Geben der Biomasse in ein Reaktionsgefäß und Inkontaktbringen der Biomasse mit einem Lösungsmittel, wobei:

i. das Verhältnis zwischen Lösungsmittel und Biomasse etwa 10:1 bis etwa 4:1 beträgt;
ii. das Lösungsmittel 40 bis 60 % w/w Ethanol enthält;
iii. der Anfangs-pH-Wert des Biomasse/Lösungsmittel-Gemischs etwa 1,5 bis etwa 2,5 beträgt;

b) Erhöhen der Temperatur des Biomasse/Lösungsmittel-Gemischs auf etwa 140°C bis etwa 170°C für eine Gesamtdauer von etwa 50 Minuten bis etwa 200 Minuten, um einen Zellstoff und eine Extraktionsbrühe zu bilden, wobei die genannte Brühe Derivate von nativem Lignin beinhaltet;
c) Gewinnen der Extraktionsbrühe;
d) Gewinnen des Zellstoffs;

wobei der Druck im Reaktionsgefäß etwa 18 bar oder weniger beträgt, und wobei Schritt a) vorbehandelte Biomassefeststoffe mit einem Time-to-Conversion-Target (TCT - Zeit-bis-Umwandlungs-Ziel) von 120 h oder weniger und einer Overall Glucose Conversion (OGC - Gesamtglucoseumwandlung) von 50 % oder mehr hervorbringt.

3. Verfahren nach Anspruch 1 oder 2, wobei der Druck im Reaktionsgefäß etwa 16 bar oder weniger beträgt.

4. Verfahren nach Anspruch 1 oder 2, wobei die erhöhte Temperatur aus Schritt (b) etwa 150°C bis etwa 165°C beträgt.

5. Verfahren nach Anspruch 1 oder 2, wobei die erhöhte Temperatur aus Schritt (b) etwa 120 Minuten bis etwa 180 Minuten lang gehalten wird.

6. Verfahren nach Anspruch 1 oder 2, wobei das Lösungsmittel 50 % bis 60 % w/w Ethanol enthält.

7. Verfahren nach Anspruch 1 oder 2, wobei etwa 2 % bis etwa 2,5 % (bezogen auf das Trockengewicht der Biomasse) einer Säure zugegeben werden.

8. Verfahren nach Anspruch 1 oder 2, wobei der pH-Wert des Biomasse/Lösungsmittel-Gemischs etwa 1,6 bis etwa 2,3 beträgt.

9. Verfahren nach Anspruch 1 oder 2, wobei das Verhältnis zwischen Lösungsmittel und Biomasse etwa 9:1 bis etwa 7:1 beträgt.

10. Verfahren nach Anspruch 1 oder 2, wobei die Biomasse Weichholzausgangsmaterial, Hartholzausgangsmaterial, Faserausgangsmaterial einjähriger Pflanzen oder eine Kombination davon beinhaltet.

11. Verfahren nach Anspruch 1 oder 2, wobei die Ausbeute des aus der Extraktionsbrühe gewonnenen Lignins bei 60 % der theoretischen Maximalausbeute oder darüber liegt.

12. Verfahren nach Anspruch 1 oder 2, wobei die Ausbeute des aus der Extraktionsbrühe gewonnenen Lignins bei 70 % der theoretischen Maximalausbeute oder darüber liegt.

13. Verfahren nach Anspruch 1 oder 2, wobei die Ausbeute des aus der Extraktionsbrühe gewonnenen Lignins bei 80 % der theoretischen Maximalausbeute oder darüber liegt.

14. Verfahren nach Anspruch 1 oder 2, wobei wenigstens ein Teil des gewonnenen Zellstoffs in Kohlenhydrate umgewandelt wird, die anschließend in Ethanol umgewandelt werden.

**Revendications**

1. Procédé organosolv pour traiter une biomasse lignocellulosique comprenant les étapes consistant à :

   a) ajouter la biomasse dans un récipient de réaction et exposer la biomasse à un solvant dans lequel :

      i. le rapport du solvant à la biomasse est compris entre environ 10:1 et environ 4:1 ;
      ii. le solvant comprend entre 40 et 60 % m/m d'éthanol ;
      iii. entre environ 1,5 % et environ 2,5 % (sur la base de la masse sèche de la biomasse) d'un acide sont ajoutés ;

   b) augmenter la température du mélange biomasse/solvant entre environ 140 °C et environ 170 °C pendant une période totale d'environ 50 minutes à environ 200 minutes pour former une pâte et une liqueur d'extraction, ladite liqueur comprenant des dérivés de lignine native ;
   c) récupérer au moins une partie de la liqueur d'extraction ;
   d) récupérer au moins une partie de la pâte ;

   dans lequel la pression dans le récipient de réaction est d'environ 18 bar ou moins, et
   dans lequel l'étape a) produit une biomasse solide prétraitée avec un temps de conversion cible (TCT) égal ou inférieur à 120 h et une conversion globale du glucose (OGC) de 50 % ou plus.

2. Procédé organosolv pour traiter une biomasse lignocellulosique comprenant les étapes consistant à :

   a) ajouter la biomasse dans un récipient de réaction et exposer la biomasse à un solvant dans lequel :

      i. le rapport du solvant à la biomasse est compris entre environ 10:1 et environ 4:1 ;
      ii. le solvant comprend entre 40 et 60 % m/m d'éthanol ;
      iii. le pH initial du mélange biomasse/solvant est compris entre environ 1,5 et environ 2,5 ;

b) augmenter la température du mélange biomasse/solvant entre environ 140 °C et environ 170 °C pendant une période totale d'environ 50 minutes à environ 200 minutes pour former une pâte et une liqueur d'extraction, ladite liqueur comprenant des dérivés de lignine native ;
c) récupérer la liqueur d'extraction ;
d) récupérer la pâte ;

dans lequel la pression dans le récipient de réaction est d'environ 18 bar ou moins, et
dans lequel l'étape a) produit une biomasse solide prétraitée avec un temps de conversion cible (TCT) égal ou inférieur à 120 h et une conversion globale du glucose (OGC) de 50 % ou plus.

3. Procédé selon la revendication 1 ou 2, dans lequel la pression dans le récipient de réaction est d'environ 16 bar ou moins.

4. Procédé selon la revendication 1 ou 2, dans lequel la température augmentée de l'étape (b) est comprise entre environ 150 °C et environ 165 °C.

5. Procédé selon la revendication 1 ou 2, dans lequel la température augmentée de l'étape (b) est maintenue pendant environ 120 minutes à environ 180 minutes.

6. Procédé selon la revendication 1 ou 2, dans lequel le solvant comprend entre 50 % et 60 % m/m d'éthanol.

7. Procédé selon la revendication 1 ou 2, dans lequel entre environ 2 % et environ 2,5 % (sur la base de la masse sèche de la biomasse) d'un acide sont ajoutés.

8. Procédé selon la revendication 1 ou 2, dans lequel le pH du mélange biomasse/solvant est compris entre environ 1,6 et environ 2,3.

9. Procédé selon la revendication 1 ou 2, dans lequel le rapport du solvant à la biomasse est compris entre environ 9:1 et environ 7:1.

10. Procédé selon la revendication 1 ou 2, dans lequel la biomasse comprend une charge d'alimentation de résineux, une charge d'alimentation de feuillus, une charge d'alimentation de fibres annuelles ou une combinaison de celles-ci.

11. Procédé selon la revendication 1 ou 2, dans lequel le rendement en lignine récupéré de la liqueur d'extraction est de 60 % ou plus du rendement maximal théorique.

12. Procédé selon la revendication 1 ou 2, dans lequel le rendement en lignine récupéré de la liqueur d'extraction est de 70 % ou plus du rendement maximal théorique.

13. Procédé selon la revendication 1 ou 2, dans lequel le rendement en lignine récupéré de la liqueur d'extraction est de 80 % ou plus du rendement maximal théorique.

14. Procédé selon la revendication 1 ou 2, dans lequel au moins une partie de la pâte récupérée est convertie en glucides qui sont ensuite convertis en éthanol.

Figure 1

## Figure 2

Figure 3 Calculation of S10005865 TCT

Figure 4 Calculation of 14(1) TCT

Y=105.36762*X/(29.03843+X); $r^2$=0.99769
TCT=121.5 h

Figure 5 Calculation of 10005636 TCT

**EP 2 588 664 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4100016 A
- US 4764596 A
- US 5681427 A
- US 7465791 B
- US 20090118477 A
- US 20090062516 A
- US 200900669550 A
- US 7649086 B
- WO 2007129921 A

**Non-patent literature cited in the description**

- **KENDALL PYE ; JAIRO H. LORA.** *The Alcell™ Process, Tappi Journal,* March 1991, 113-117